# FASCICULE DE BREVET EUROPEEN

(11) **EP 0 751 788 B1**
(45) Date de publication et mention de la délivrance du brevet: **29.12.1999**
(21) Numéro de dépôt: 95914392.6
(22) Date de dépôt: 21.03.1995
(51) Int. Cl.: A61K 39/39, A61K 48/00

(54) **UNE COMPOSITION COMPRENANT UN PLASMIDE RECOMBINANT ET SES UTILISATIONS COMME VACCIN ET MEDICAMENT**
REKOMBINANTE PLASMIDE ENTHALTENDE ZUSAMMENSETZUNG UND IHRE VERWENDUNG ALS IMPSTOFF UND ARZNEIMITTEL
COMPOSITION INCLUDING A RECOMBINANT PLASMID, AND USES THEREOF AS A VACCINE AND DRUG

(30) Priorité: 22.03.1994 FR 9403361
(43) Date de publication de la demande: 08.01.1997
(73) Titulaire: S.E.P.P.I.C., SOCIETE D'EXPLOITATION DE PRODUITS POUR LES INDUSTRIES CHIMIQUES, F-75007 Paris (FR)
(72) Inventeur: GANNE, Vincent, F-94210 La-Varenne-Saint-Hilaire (FR)
(74) Mandataire: Le Moenner, Gabriel
(86) Numéro de dépôt international: FR9500345
(87) Numéro de publication internationale: WO9525542

(56) Documents cités:
- WO-A-91/00106
- WO-A-94/16681
- CIRCULATION, vol. 82, no. 6, Décembre 1990 pages 2217-2221, LIN H. ET AL. 'Expression of Recombinant genes in Myocardium In Vivo After Direct Injection of DNA'
- JOURNAL OF VIROLOGY, vol. 67, no. 9, Septembre 1993 WASHINGTON (US), pages 5664-5667, COX G.J.M. ET AL. 'Bovine Herpesvirus 1: Immune Responses in Mice and Cattle Injected with Plasmid DNA'

## Description

La présente invention concerne une composition comprenant un plasmide recombinant, ainsi que des vaccins ou des médicaments curatifs comprenant une telle composition.

La vaccination par des virus ou des micro-organismes vivants présente de nombreux avantages bien connus comparée à la vaccination par des vaccins non vivants constitués de micro-organismes tués ou de protéines ou peptides isolé(e)s.

Les vaccins vivants sont constitués d'un micro-organisme ou d'un virus vivants. Ces derniers sont généralement atténués, de sorte à diminuer les risques pathogènes. Cependant, ce risque n'est jamais totalement écarté dans la mesure où, notamment, une réversion du micro-organisme ou du virus vers une forme virulente peut survenir.

Par ailleurs, les mutations ou délétions mises en oeuvre pour conduire à une atténuation de la virulence des souches entraînent parfois une diminution de la réponse immunitaire, nécessitant l'injection de doses importantes de vaccins. Récemment, de nouveaux vaccins dont la préparation a été rendue possible par les progrès du génie génétique, ont été mis au point. Parmi ces nouveaux vaccins, on cite ceux consistant en un plasmide recombinant constitué d'une séquence de nucléotides dans laquelle est insérée une séquence nucléotidique exogène provenant d'un micro-organisme ou d'un virus pathogène. Cette dernière séquence nucléotidique a pour but de permettre l'expression d'un composé comprenant une séquence d'acides aminés, ce composé ayant lui-même pour but de déclencher une réaction immune dans un organisme hôte.

La première injection d'un tel plasmide ainsi que son expression dans un muscle a été effectuée et démontrée en 1990 par Lin et al (Circulation 82:2217-2221). Cette expérimentation avait pour but de démontrer qu'un plasmide recombinant injecté dans un organisme vivant était capable d'exprimer la séquence exogène dans les tissus dans lesquels il avait été injecté. Il a ainsi été démontré que l'administration dans un organisme vivant d'un plasmide recombinant pourrait être utilisée en thérapie génique. Cette méthode thérapeutique est bien connue et consiste notamment à administrer dans un organisme hôte des cellules génétiquement modifiées, ou, comme l'ont démontré Lin et al des plasmides recombinants capables d'exprimer des composés synthétisés par des organismes vivants, tels des peptides, des protéines ou des glycoprotéines. La synthèse in vivo de ces composés peut avoir pour but soit de suppléer un déficit d'ordre génétique de l'organisme hôte dans lequel les cellules génétiquement modifiées ou les plasmides recombinants ont été administré(es), soit d'avoir une action curative à l'encontre d'une maladie, telle un cancer, déclenché chez ce même organisme hôte.

A titre d'exemple, une telle action curative peut consister en une synthèse, par des cellules recombinantes ou des plasmides recombinants, de cytokines telles des interleukines, notamment l'interleukine 2. Celles-ci permettent le déclenchement ou le renforcement d'une réaction immune visant à l'élimination sélective des cellules cancéreuses.

Ultérieurement, il a été montré que des plasmides recombinants portant un gène spécifique, codant pour la glycoprotéine de l'herpesvirus I bovin (BHV-1), étaient capables d'induire une réponse immune, consistant en la synthèse d'anticorps, dans différentes espèces animales, à savoir les souris et les bovins (Cox et al, J.Virol., septembre 1993, 67, 9, 5664, 5667). Cependant, cette étude montre aussi de manière claire une hétérogénéité des réponses immunitaires obtenues. Autrement dit, toutes conditions étant égales par ailleurs, la réponse immunitaire est très variable pour chaque animal testé et chaque injection effectuée. Il en ressort que quelques animaux présentent un taux d'anticorps faible, bien que parfois suffisant pour induire une certaine protection, alors que la plupart des autres animaux ne présentent pas un tel taux d'anticorps. Dans ce dernier cas, aucune protection n'est induite.

La présente invention consiste alors en une composition comprenant un plasmide recombinant capable d'exprimer chez un organisme hâte dans lequel ledit plasmide recombinant a été administré, une quantité plus importante de composés du type peptides, protéines ou glycoprotéines.

Un autre objet de l'invention consiste en une composition contenant un plasmide recombinant en une concentration inférieure à celle des compositions classiques, ce en vue d'une expression de composés du type peptides, protéines ou glycoprotéines, en quantité inchangée, voire supérieure.

Un autre objet de l'invention consiste en un vaccin comprenant une composition contenant un plasmide recombinant permettant l'obtention d'une réponse immunitaire plus homogène et d'une protection renforcée contre un virus ou un micro-organisme pathogène.

Encore un autre objet de l'invention consiste en un médicament curatif comprenant une composition contenant un plasmide recombinant capable d'exprimer des quantités accrues de molécules du type peptides, protéines ou glycoprotéines, et d'être mis en oeuvre avec une concentration réduite en plasmides recombinants.

L'invention consiste ainsi en une composition contenant un plasmide recombinant comprenant une séquence nucléotidique exogène capable d'exprimer dans un organisme hôte un composé comprenant une séquence d'acides aminés, caractérisée en ce que ladite composition comprend une émulsion comportant au moins une phase aqueuse et au moins une phase huile, ledit plasmide recombinant étant contenu dans l'une au moins desdites phases.

La figure représente les résultats d'un test de résistance aux challenge effectué sur des souris auxquelles on a administré soit un plasmide recombinant portant le gène GP50 du virus Aujeszky, soit divers témoins.

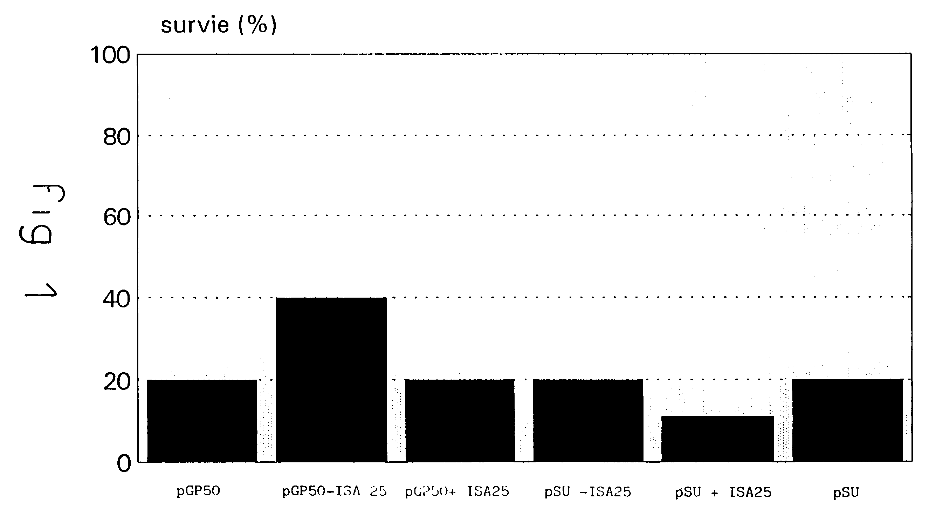

Un plasmide recombinant selon l'invention peut être préparé selon des méthodes classiques, par exemple la méthode décrite dans l'article de Cox et al, J. Virol, septembre 1993, 67, 9, 5664-5667, mentionné ci-dessus ou dans l'article de M. Eloit et al, J. of General Virol (1990), 71, 2425-2431 (en particulier la figure 1).

Bien entendu, la séquence nucléotidique exogène varie en fonction du composé à exprimer.

Le composé pouvant être exprimé par le plasmide recombinant lorsqu'il a été administré à l'organisme hôte, peut être un peptide, une protéine, ou une glycoprotéine.

Ce composé peut notamment consister en un antigène capable de déclencher dans ledit organisme hôte une réaction immune vis-à-vis d'un virus pathogène ou d'un micro-organisme pathogène. Cette réaction immune peut être de type humoral ou cellulaire et notamment consister en une synthèse d'anticorps permettant de conférer une protection à l'organisme hôte vis-à-vis d'un tel virus ou d'un tel micro-organisme. A titre de tels virus pathogènes, on peut notamment citer le virus Aujesky, un virus HIV comme le HIV-I ou le HIV-II, un virus FIV ou un virus de la grippe du type Influenzae. Dans le cadre de la présente invention, on entend par micro-organisme, une bactérie, une levure, un champignon, un mycoplasme ou un parasite unicellulaire. A titre de bactéries pathogènes, on peut notamment citer les furonculoses, Escherichia coli ou des bactéries des genres Pastorella, Salmonella ou Yersinia. A titre de levures pathogènes, on peut citer celles du genre Candida, comme Candida albicans. A titre de parasites unicellulaires, on peut citer Plasmodium falciparum ou les parasites du genre Leishmania.

Habituellement, la séquence nucléotidique exogène provient d'un tel virus ou micro-organisme pathogène.

Alternativement, le plasmide recombinant administré dans l'hôte d'accueil peut comprendre une séquence nucléotidique capable d'exprimer un composé comprenant une séquence d'acides aminés, comme un peptide, une protéine ou une glycoprotéine, ayant une action curative vis-à-vis d'une maladie, en particulier d'une maladie non infectieuse pouvant être d'ordre fonctionnel, qui s'est déclenchée chez l'organisme hôte. Dans ce cas, le plasmide recombinant permet un traitement thérapeutique du type thérapie génique, telle que définie plus haut. Ainsi, ledit composé peut avoir pour fonction de suppléer à un déficit d'ordre génétique de l'organisme hôte, en vue de traiter une maladie génétique comme la mucoviscidose ou la myopathie. Ledit composé peut également avoir une action curative vis-à-vis d'une maladie fonctionnelle qui s'est déclarée chez l'organisme hôte ; par exemple, il peut avoir une action curative vis-à-vis des cellules cancéreuses. Cette action curative peut consister en la synthèse de cytokines, commes les interleukines, telle l'interleukine-2.

L'organisme hôte dans lequel ledit plasmide recombinant doit être capable d'exprimer un composé comprenant une séquence d'acides aminés peut être un animal, voire un tissu d'un animal, comme un insecte ou un animal vertébré, en particulier un mammifère, un poisson ou un oiseau. Un tel mammifère peut être l'homme, un canidé, un bovidé, un porc, un lapin, un ovidé ou un félidé. A titre d'oiseau, on peut citer les gallinacés comme les poulets.

Une composition selon l'invention peut comporter une émulsion du type huile dans eau (H/E), eau dans huile (E/H) ou eau dans huile dans eau (E/H/E). Un type d'émulsion particulièrement préféré dans le cadre de l'invention consiste en une émulsion H/E. Une émulsion selon l'invention peut être préparée selon les méthodes classiques de préparation d'une émulsion, notamment selon les procédés décrits dans les demandes de brevets EP-A-489.181 et EP-A-481.982. Ainsi, on peut émulsionner sous agitation l'huile constitutive de la phase huile avec la phase aqueuse constituée d'une solution ou d'une suspension aqueuse contenant le plasmide recombinant. Alternativement, on peut préparer la composition de l'invention en émulsionnant une huile comprenant le plasmide recombinant, par exemple sous forme lyophilisée, avec une phase aqueuse.

Une émulsion selon l'invention peut comporter, en poids, de 5 à 95 % de phase huile pour 95 à 5 % de phase aqueuse et, de préférence, de 25 à 75 % de phase huile pour 75 à 25 % de phase aqueuse. L'émulsion doit être stable de préférence pendant au moins 12 mois quand elle est stockée à 4°C.

L'huile constitutive de la phase huile peut être une huile minérale, une huile non minérale ou un mélange d'une huile minérale et d'une huile non minérale. Lesdites huiles minérales peuvent être naturelles ou synthétiques. Lesdites huiles non minérales peuvent être d'origine végétale, animale ou synthétique. Toutes ces huiles sont dépourvues d'effets toxiques à l'égard de l'organisme hôte chez lequel la composition de l'invention est administrée. Elles sont de préférence liquides à la température de stockage (environ +4°C) ou au moins permettre de donner des émulsions liquides à cette température. Une huile minérale avantageuse selon l'invention peut consister en une huile comprenant une chaîne carbonée linéaire ayant un nombre d'atomes de carbone de préférence supérieur à 16, et exempte de composés aromatiques. De telles huiles peuvent être par exemple celles commercialisées sous la désignation "MARCOL® 52" (produit par Esso France) ou "DRAKEOL 6VR® (produite par Penreco USA).

A titre d'huiles organiques synthétiques, on peut citer les polyisobutènes ou les polyisopropènes. Parmi les huiles végétales, on peut citer des huiles insaturées riches en acide oléique qui sont biodégradables, par exemple les huiles d'arachide, d'olive, de sésame, de soja ou de germes de blé.

Les huiles animales peuvent consister notamment en le squalène, le squalane ou l'huile de spermacéti.

Outre la phase huile et la phase aqueuse, la composition selon l'invention, notamment quand elle est utilisée comme vaccin, peut comporter un agent stimulant immunitaire tel l'avridine.

Par ailleurs, la composition selon l'invention peut également avantageusement comporter un agent tensio-actif. Ce dernier présente un caractère lipophile ou hydrophile caractérisé par une valeur HLB (hydrophile-lipophile-balance) comprise entre 1 et 19.

Un agent tensio-actif préféré dans le cadre de la présente invention peut consister en un ester obtenu par condensation d'un acide gras, avantageusement un acide gras liquide à 20°C, avec un sucre ou du glycérol. Ledit sucre peut consister en le glucose, le saccharose ou, de préférence, le mannitol. A titre d'ester de mannitol particulièrement préféré, on peut citer des oléates de mannitol obtenus par anhydridation de la chaîne carbonée polyhydroxylée du mannitol qui se cyclise en 1-4 ou en 2-6.

Des dérivés de ces esters peuvent être également mis en oeuvre. Ces dérivés présentent une hydrophilie modifiée notamment par greffage de fonction hydrophiles telles alcool, polyol, oxyde d'éthylène, oxyde de propylène, acide carboxylique, amine, ou amide. Un agent tensio-actif selon l'invention est de préférence pharmaceutiquement acceptable pour un usage en injectable ; il doit être notamment dépourvu de métaux lourds et présenter des indices d'acides ou de peroxydes très faibles. Il est également souhaitable qu'il satisfasse les normes de test d'innocuité tel que par exemple, celui décrit par S.S. Berlin, Annales of Allergy, 1962, 20, 473. Préférentiellement, l'agent tensio-actif est associé à l'huile avant formation de l'émulsion.

Des huiles associées avec un agent tensio-actif convenant tout particulièrement dans le cadre de la présente invention sont celles commercialisées par la Société SEPPIC sous la marque "MONTANIDE®". Les caractéristiques de ces huiles figurent dans le tableau 1 ci-dessous.

**Tableau 1**

| N° | Nom commercial | Huile | Type de l'émulsion | Phase aqueuse/ émulsion (% en poids) | Viscosité (m.Pa.s) | Conductivité à 25°C (µS.cm⁻¹) |
|---|---|---|---|---|---|---|
| 1 | MONTANIDE ISA 25 | Minérale | H/E | 75 % | 20 | 5000 |
| 2 | MONTANIDE ISA 25A | Minérale + avridine* | H/E | 75 % | 20 | 5000 |
| 3 | MONTANIDE ISA 28 | Minérale + non minérale | H/E | 75 % | 25 | 1000 |
| 4 | MONTANIDE ISA 206 | Minérale | E/H/E | 50 % | 50 | 1000 |
| 5 | MONTANIDE ISA 50 | Minérale | E/H | 50 % | 200 | 1 |
| 6 | MONTANIDE ISA 708 | Non minérale | E/H | 30 % | 70 | 1 |

| | | | | | | |
|---|---|---|---|---|---|---|
| * Avridine = N,N-dioctadecyl-N-bis-(2-hydroxyéthyl)-propanediamine. | | | | | | |

Une composition selon l'invention présente une viscosité inférieure à 300 mPa.s, avantageusement inférieure à 200 m.Pa.s, mesurée à 25°C au moyen d'un viscosimètre à mobile tournant du type Brookfield. Le caractère "huileux" ou "aqueux" de la phase continue de l'émulsion est caractérisée par la conductivité mesurée en micro-Siemens par cm (µS.cm⁻¹) à 25°C. Des valeurs inférieures à 20 µS.cm⁻¹ indiquent la présence d'une phase huile continue.

Une composition selon l'invention comprend une quantité de plasmide recombinant variable en fonction, principalement, de l'organisme hôte et de la nature du composé exprimé par ledit plasmide.

Habituellement, une composition selon l'invention peut comprendre de 0,01 à 100 g/l dudit plasmide recombinant.

Selon un autre aspect, l'invention concerne également un vaccin comprenant une composition telle que définie ci-dessus. Un tel vaccin a pour but de provoquer une réaction immune à l'encontre d'un virus ou d'un micro-organisme pathogène, tels ceux cités plus haut.

Selon encore un autre aspect, l'invention concerne un médicament curatif comprenant une composition telle que définie ci-dessous. Un tel médicament a pour but de soigner une maladie, notamment une maladie fonctionnelle de l'organisme hôte dans lequel il est administré.

Ces vaccins et médicaments curatifs comprenant ladite composition se présentent généralement sous une forme injectable. L'organisme hôte dans lequel ils peuvent être administrés sont les animaux, voire des tissus d'animaux cités plus haut. On mentionnera ici plus particulièrement l'homme, le chien, le chat, la volaille, le boeuf, le mouton, le porc et le cheval.

La dose de plasmide recombinant contenu dans ladite composition à administrer dépend de la nature de l'organisme hâte. Cette dose de plasmide recombinant peut alors varier très largement entre 1 µg et 500 mg/kg de l'organisme hôte.

A titre d'exemple, on peut administrer une dose de plasmide recombinant de 500 mg/kg à une souris de 20 g, de 1 µg/kg à un boeuf de 500 kg et une dose de 100 µg à 100 mg/kg, de préférence de 1 mg à 30 mg/kg, chez l'homme adulte.

Une composition selon l'invention peut être plus particulièrement utilisée pour préparer un vaccin destiné à produire une réaction immune chez un organisme hôte, pour la prévention à l'encontre d'un virus HIV (notamment chez l'homme), d'un virus Aujeszky (notamment chez le porc) ou d'un virus FIV (notamment chez le chat).

Une composition selon l'invention peut encore être utilisée pour préparer un médicament destiné à soigner un cancer..

Les exemples qui suivent ont pour but d'illustrer la présente invention.

Tous ces exemples mettent en oeuvre un plasmide recombinant portant le gène gp50 du virus Aujeszky. Celui-ci a été obtenu selon la méthode décrite dans l'article de M. Eloit et al, J. of General Virol (1990), 71, 2425-2431 (en particulier la figure 1) mentionné plus haut.

Dans ces exemples, les termes suivants signifient :
- pGP50-ISA 25 (conforme à l'invention) : un vaccin comportant ledit plasmide recombinant portant le gène GP50 du virus Aujeszky dans une émulsion comportant, en tant que phase huile, le MONTANIDE® ISA 25 (cf. tableau 1) ;
- pGP50 + ISA 25 (non conforme à l'invention): un vaccin injecté en deux temps: dans un premier temps, on injecte une suspension aqueuse comportant ledit plasmide recombinant portant le gène GP50 du virus Aujeszky et, dans un second temps, on injecte une émulsion (ne comportant pas de plasmide) comportant le MONTANIDE® ISA 25;
- pGP50 (non conforme à l'invention) : un vaccin constitué uniquement d'une phase aqueuse comprenant le plasmide recombinant ;
- pSU-ISA 25 (témoin): un vaccin similaire à pGP50-ISA 25 mais comprenant un plasmide témoin exprimant une protéine exogène pSU, différente de pGP50 ;
- pSU + ISA 25 (témoin) : un vaccin similaire à pGP50-ISA 25 mais comprenant un plasmide témoin exprimant pSU au lieu de pGP50 ;
- pSU : un vaccin similaire à pGP50 mais comprenant un plasmide témoin exprimant pSU au lieu de pGP50.

### Exemple 1 :

On injecte à des souris numérotées 1 à 6 différents vaccins.

A intervalles réguliers, on dose les anticorps produits par ces vaccins. Pour ce faire, on prélève le sérum des souris et on dose les anticorps anti-GP50 par une technique ELISA décrite par M. Eloit et al dans Veterinary Record, 1989, 124, 91-94. Les résultats figurent dans les tableaux 2 à 5 ci-après. Ils sont exprimés en fonction de la dernière dilution nécessaire à l'obtention d'une densité optique supérieure au bruit de fond.

Les résultats obtenus montrent qu'un vaccin selon l'invention permet l'obtention d'un taux d'anticorps nettement supérieur à celui obtenu avec les vaccins comparatifs ou les témoins, ainsi qu'une plus forte homogénéité de la réponse immunitaire. Cette homogénéité se traduit par l'obtention d'un taux d'anticorps élevé, permettant l'induction d'une protection efficace chez un nombre plus important d'animaux.

**Tableau 2**

| Titrage des anticorps anti-GP50 par dosage ELISA 2 semaines après vaccination : | | | | | | |
|---|---|---|---|---|---|---|
| **Vaccin** | **souris 1** | **souris 2** | **souris 3** | **souris 4** | **souris 5** | **souris 6** |
| pGP50-ISA 25 | 32 | 1024 | 32 | 1024 | - | - |
| pGP50 +ISA 25 | 16 | 16 | 32 | 16 | - | - |
| pGP50 | 16 | 8 | 8 | 16 | 256 | 16 |
| pSU-ISA 25 | < 8 | < 8 | < 8 | < 8 | - | - |
| pSU + ISA 25 | < 8 | < 8 | < 8 | < 8 | - | - |
| pSU | < 8 | < 8 | < 8 | < 8 | - | - |

**Tableau 3**

| Titrage des anticorps anti-GP50 par dosage ELISA 4 semaines après vaccination : | | | | | | |
|---|---|---|---|---|---|---|
| **Vaccin** | **souris 1** | **souris 2** | **souris 3** | **souris 4** | **souris 5** | **souris 6** |
| pGP50 - ISA 25 | 32 | 384 | 32 | 1024 | - | - |
| pGP50 + ISA 25 | 16 | 8 | 8 | 8 | - | - |
| pGP50 | 16 | 8 | 8 | 16 | 128 | 8 |
| pSU - ISA 25 | < 8 | < 8 | < 8 | < 8 | - | - |
| pSU + ISA 25 | < 8 | < 8 | < 8 | < 8 | - | - |
| pSU | < 8 | < 8 | < 8 | < 8 | - | - |

**Tableau 4**

| Titrage des anticorps anti-GP50 par dosage ELISA 6 semaines après vaccination : | | | | | | |
|---|---|---|---|---|---|---|
| **Vaccin** | **souris 1** | **souris 2** | **souris 3** | **souris 4** | **souris 5** | **souris 6** |
| pGP50 - ISA 25 | 1536 | 2048 | - | 2048 | - | - |
| pGP50 + ISA 25 | 128 | 128 | 128 | 48 | - | - |
| pGP50 | 384 | 64 | 256 | 512 | 128 | 128 |
| pSU - ISA 25 | < 8 | < 8 | < 8 | < 8 | - | - |
| pSU + ISA 25 | < 8 | < 8 | < 8 | < 8 | - | - |
| pSU | < 8 | < 8 | < 8 | < 8 | - | - |

**Tableau 5**

| Titrage des anticorps anti-GP50 par dosage ELISA 3 mois après vaccination : | | | | | | |
|---|---|---|---|---|---|---|
| **Vaccin** | **souris 1** | **souris 2** | **souris 3** | **souris 4** | **souris 5** | **souris 6** |
| pGP50 - ISA 25 | 2048 | 1280 | - | 768 | - | - |
| pGP50 + ISA 25 | 256 | 256 | 256 | 256 | - | - |
| pGP50 | 384 | 128 | 256 | 256 | 192 | 256 |
| pSU - ISA 25 | < 8 | < 8 | < 8 | < 8 | - | - |
| pSU + ISA 25 | < 8 | < 8 | < 8 | < 8 | - | - |
| pSU | < 8 | < 8 | < 8 | < 8 | - | - |

### Exemple 2 :

On vaccine 6 groupes de 6 souris chacun avec un vaccin différent :
- pGP 50,
- pGP50 - ISA 25,
- pGP50 + ISA 25,
- pSU - ISA 25,
- PSU + ISA 25, et
- pSU.

Six semaines après l'injection du vaccin, on injecte à chaque souris une suspension aqueuse contenant un virus Aujeszky virulent.

Le pourcentage de survie des souris après cette dernière injection est alors déterminé.

Les résultats obtenus sont présentés sur la figure où le taux de survie est en ordonnée. Ces résultats montrent qu'un vaccin selon l'invention pGP50 - ISA 25 permet de conférer une protection deux fois plus importante qu'avec un vaccin de l'art antérieur comprenant seulement un plasmide recombinant pGP50 en suspension aqueuse.

## Revendications

1. Une composition contenant un plasmide recombinant comprenant une séquence nucléotidique exogène capable d'exprimer dans un organisme hôte un composé comprenant une séquence d'acides aminés, caractérisée en ce que ladite composition comprend une émulsion comportant au moins une phase aqueuse et au moins une phase huile, ledit plasmide recombinant étant contenu dans l'une au moins desdites phases.

2. Une composition selon la revendication 1, caractérisée en ce que ledit composé consiste en une protéine, une glycoprotéine ou un peptide.

3. Une composition selon l'une des revendications 1 et 2, caractérisée en ce que ledit composé consiste en un antigène capable de déclencher dans l'organisme hôte, une réaction immune vis-à-vis d'un virus pathogène ou d'un micro-organisme pathogène.

4. Une composition selon la revendication 3, caractérisée en ce que le virus pathogène est le virus Aujeszky, un virus HIV ou un virus FIV.

5. Une composition selon la revendication 2, caractérisée en ce que ledit composé est la glycoprotéine gp50 du virus Aujeszky.

6. Une composition selon l'une des revendications 1 et 2, caractérisée en ce que ledit composé a une action curative vis-à-vis d'une maladie de l'organisme hôte.

7. Une composition selon la revendication 6, caractérisée en ce que ledit composé supplée à un déficit d'ordre génétique de l'organisme hôte.

8. Une composition selon l'une des revendications 1 et 2, caractérisée en ce que ledit composé a une action curative vis-à-vis des cellules cancéreuses présentes dans l'organisme hôte.

9. Une composition selon l'une des revendications 1 à 8, caractérisée en ce que l'organisme hôte est un animal vertébré ou un insecte.

10. Une composition selon la revendication 9, caractérisée en ce que l'animal vertébré est un mammifère ou un oiseau.

11. Une composition selon la revendication 10, caractérisée en ce que le mammifère est l'homme.

12. Une composition selon l'une des revendications 1 à 11, caractérisée en ce que l'émulsion est du type H/E, E/H ou E/H/E.

13. Une composition selon la revendication 12, caractérisée en ce que l'émulsion est du type H/E.

14. Une composition selon l'une des revendications 1 à 13, caractérisée en ce que l'émulsion comporte en outre au moins un agent tensio-actif, de préférence un agent tensio-actif consistant en un ester obtenu par condensation d'un acide gras avec un sucre ou du glycérol, ou un dérivé d'un tel ester dont l'hydrophilie a été modifiée et de préférence les oléates de mannitol modifiés par anhydridation de la chaîne carbonée polyhydroxylée du mannitol.

15. Une composition selon l'une des revendications 1 à 14, caractérisée en ce qu'elle présente une viscosité inférieure à 300 mPa.s à 25°C.

16. Un vaccin, caractérisé en ce qu'il comprend une composition selon l'une des revendications 1 à 15.

17. Un médicament curatif, caractérisé en ce qu'il comprend une composition selon l'une des revendications 1 à 15.

18. Utilisation d'une composition selon l'une des revendications 1 à 15 pour la préparation d'un vaccin destiné à produire une réaction immunitaire à l'encontre d'un virus HIV, du virus Aujeszky ou du virus FIV chez un organisme hôte.

19. Utilisation d'une composition selon l'une des revendications 1 à 15 pour la préparation d'un médicament destiné à soigner un cancer.

## Claims

1. Composition containing a recombinant plasmid comprising an exogenous nucleotide sequence capable of expressing a compound comprising an amino acid sequence in a host body, characterized in that the said composition comprises an emulsion containing at least one aqueous phase and at least one oil phase, the said recombinant plasmid being contained in at least one of the said phases.

2. Composition according to Claim 1, characterized in that the said compound consists of a protein, a glycoprotein or a peptide.

3. Composition according to either of Claims 1 and 2, characterized in that the said compound consists of an antigen capable of triggering in the host body an immune reaction with respect to a pathogenic virus or to a pathogenic microorganism.

4. Composition according to Claim 3, characterized in that the pathogenic virus is the Aujeszky virus, an HIV virus or an FIV virus.

5. Composition according to Claim 2, characterized in that the said compound is the gp50 glycoprotein of the Aujeszky virus.

6. Composition according to either of Claims 1 and 2, characterized in that the said compound has a curative action with respect to a disease of the host body.

7. Composition according to claim 6, characterized in that the said compound compensates for a deficiency of a genetic nature in the host body.

8. Composition according to either of Claims 1 and 2, characterized in that the said compound has a curative action with respect to cancer cells present in the host body.

9. Composition according to one of Claims 1 to 8, characterized in that the host body is a vertebrate animal or an insect.

10. Composition according to Claim 9, characterized in that the vertebrate animal is a mammal or a bird.

11. Composition according to Claim 10, characterized in that the mammal is man.

12. Composition according to one of Claims 1 to 11, characterized in that the emulsion is of O/W, W/O or W/O/W type.

13. Composition according to Claim 12, characterized in that the emulsion is of the O/W type.

14. Composition according to one of Claims 1 to 13, characterized in that the emulsion contains, in addition, at least one surfactant, preferably a surfactant consisting of an ester obtained by condensing a fatty acid with a sugar or glycerol, or a derivative of such an ester whose hydrophilicity has been modified and preferably mannitol oleates modified by anhydration of the mannitol polyhydroxylated carbon chain.

15. Composition according to one of Claims 1 to 14, characterized in that it possesses a viscosity of less than 300 mPa.s at 25°C.

16. Vaccine, characterized in that it comprises a composition according to one of claims 1 to 15.

17. Curative medicament, characterized in that it comprises a composition according to one of Claims 1 to 15.

18. Use of a composition according to one of Claims 1 to 15, for the preparation of a vaccine intended to produce an immune reaction against an HIV virus, the Aujeszky virus or the FIV virus in a host body.

19. Use of a composition according to one of Claims 1 to 15, for the preparation of a medicament intended to treat a cancer.

## Patentansprüche

1. Zusammensetzung mit einem rekombinanten Plasmid, umfassend eine exogene Nucleotidsequenz, die fähig ist, in einem Wirtsorganismus eine Verbindung, die eine Aminosäuresequenz umfaßt, zu exprimieren, dadurch gekennzeichnet, daß diese Zusammensetzung eine Emulsion mit mindestens einer wäßrigen Phase und mindestens einer Ölphase umfaßt, wobei das rekombinante Plasmid in mindestens einer dieser Phasen enthalten ist.

2. Zusammensetzung nach Anspruch 1, dadurch gekennzeichnet, daß die Verbindung aus einem Protein, einem Glycoprotein oder einem Peptid besteht.

3. Zusammensetzung nach Anspruch 1 oder 2, dadurch gekennzeichnet, daß die Verbindung aus einem Antigen besteht, das fähig ist, im Wirtsorganismus eine Immunreaktion gegen ein pathogenes Virus oder einen pathogenen Mikroorganismus auszulösen.

4. Zusammensetzung nach Anspruch 3, dadurch gekennzeichnet, daß es sich bei dem pathogenen Virus um den Aujeszky-Virus, ein HIV-Virus oder ein FIV-Virus handelt.

5. Zusammensetzung nach Anspruch 2, dadurch gekennzeichnet, daß es sich bei der Verbindung um das gp50-Glycoprotein des Aujeszky-Virus handelt.

6. Zusammensetzung nach Anspruch 1 oder 2, dadurch gekennzeichnet, daß die Verbindung eine kurative Wirkung auf eine Krankheit des Wirtsorganismus ausübt.

7. Zusammensetzung nach Anspruch 6, dadurch gekennzeichnet, daß die Verbindung eine genetische Defizienz des Wirtsorganismus kompensiert.

8. Zusammensetzung nach Anspruch 1 oder 2, dadurch gekennzeichnet, daß die Verbindung eine kurative Wirkung gegenüber Krebszellen, die in dem Wirtsorganismus vorliegen, ausübt.

9. Zusammensetzung nach einem der Ansprüche 1 bis 8, dadurch gekennzeichnet, daß es sich bei dem Wirtsorganismus um ein Wirbeltier oder um ein Insekt handelt.

10. Zusammensetzung nach Anspruch 9, dadurch gekennzeichnet, daß es sich bei dem Wirbeltier um ein Säugetier oder einen Vogel handelt.

11. Zusammensetzung nach Anspruch 10, dadurch gekennzeichnet, daß es sich bei dem Säugetier um den Menschen handelt.

12. Zusammensetzung nach einem der Ansprüche 1 bis 11, dadurch gekennzeichnet, daß es sich bei der Emulsion um eine O/W-, W/O- oder W/O/W-Emulsion handelt.

13. Zusammensetzung nach Anspruch 12, dadurch gekennzeichnet, daß es sich bei der Emulsion um eine O/W-Emulsion handelt.

14. Zusammensetzung nach einem der Ansprüche 1 bis 13, dadurch gekennzeichnet, daß die Emulsion außerdem mindestens ein Tensid enthält, vorzugsweise ein Tensid, das aus einem Ester besteht, der durch Kondensation einer Fettsäure mit einem Zucker oder Glycerin erhalten wurde, oder aus einem Derivat eines solchen Esters, dessen Hydrophilie modifiziert wurde, vorzugsweise Mannitoleate, die dadurch modifiziert wurden, daß man der polyhydroxylierten Kohlenstoffkette des Mannits Wasser entzieht.

15. Zusammensetzung nach einem der Ansprüche 1 bis 14, dadurch gekennzeichnet, daß sie bei 25°C eine Viskosität von unter 300mPa.s aufweist.

16. Impfstoff, dadurch gekennzeichnet, daß er eine Zusammensetzung nach einem der Ansprüche 1 bis 15 enthält.

17. Kuratives Arzneimittel, dadurch gekennzeichnet, daß es eine Zusammensetzung nach einem der Ansprüche 1 bis 15 enthält.

18. Verwendung einer Zusammensetzung nach einem der Ansprüche 1 bis 15 zur Herstellung eines Impfstoffs, der, wenn der Wirtsorganismus einem HIV-Virus, dem Aujeszky-Virus oder dem FIV-Virus ausgesetzt ist, eine Immunreaktion hervorrufen soll.

19. Verwendung einer Zusammensetzung nach einem der Ansprüche 1 bis 15 zur Herstellung eines Arzneimittels, das einen Krebs bekämpfen soll.
